# EUROPÄISCHE PATENTSCHRIFT

(11) **EP 0 966 256 B1**
(45) Veröffentlichungstag und Bekanntmachung des Hinweises auf die Patenterteilung: **06.02.2002**
(21) Anmeldenummer: 98916900.8
(22) Anmeldetag: 07.03.1998
(51) Int. Cl.: A61K 7/20, A61K 7/16, A61K 7/22

(54) **MITTEL ZUR FÖRDERUNG DER MUNDHYGIENE UND DER MUNDGESUNDHEIT**
AGENTS FOR PROMOTING ORAL HYGIENE AND ORAL HEALTH
AGENTS FAVORISANT L'HYGIENE ET LA SANTE BUCCALES

(30) Priorität: 12.03.1997 DE 19710068
(43) Veröffentlichungstag der Anmeldung: 29.12.1999
(73) Patentinhaber: Kramer, Axel, Prof.Dr.med.habil., 17489 Greifswald (DE)
(72) Erfinder: KRAMER, Axel, D-17489 Greifswald (DE); ROSIN, Michael, D-17491 Greifswald (DE)
(74) Vertreter: Jönsson, Hans-Peter, Dr.Dipl.-Chem.
(86) Internationale Anmeldenummer: EP9801340
(87) Internationale Veröffentlichungsnummer: WO9840047

(56) Entgegenhaltungen:
- WO-A-87/07838
- WO-A-96/02624
- WO-A-97/07777
- WO-A-97/26908
- CHEMICAL ABSTRACTS, vol. 98, no. 24, 13.Juni 1983 Columbus, Ohio, US; abstract no. 204403, XP002070116 & SE 425 593 A (BJORK K. E. L.) 18.Oktober 1982

## Beschreibung

Die Erfindung betrifft Mittel zur Förderung der Mundhygiene und der Mundgesundheit, insbesondere zur Hemmung der Akkumulation bakterieller Plaque auf Zähnen, Zahnersatz und jeweils angrenzenden Oberflächen zur Prophylaxe sowie zur Therapie von Entzündungen und Erkrankungen der Mundschleimhaut, der Gingiva, des Parodontiums sowie zur Kariesprophylaxe.

Zur Prophylaxe und Therapie von Munderkrankungen, insbesondere entzündlicher Erkrankungen des Parodontiums sowie der Karies werden eine Vielzahl antibiotischer, antiseptischer und antiphlogistischer Mittel eingesetzt. Der Einsatz von Antibiotika (auch der lokale Einsatz, da ein Großteil der Mittel durch Verschlucken oral aufgenommen wird) bedeutet einen radikalen Eingriff in die physiologische Bakterienflora des Verdauungstraktes einschließlich der Mundhöhle. Das gleiche gilt für Antiseptika, die in dieser Indikation ausschließlich lokal, d.h. in der Mundhöhle angewendet werden. Für antiphlogistische Mittel gilt, daß diese lediglich erkranktes Gewebe beeinflussen können, jedoch keinerlei Ansatz für die Prophylaxe oraler Erkrankungen bieten. Der Einsatz unphysiologischer Substanzen wie antibiotischer, antiseptischer und antiphlogistischer Mittel birgt zudem alle Risiken bekannter Arzneimittel-Nebenwirkungen, einschließlich der mikrobiellen Resistenzentwicklung durch Antibiotika. Es ist bekannt, daß eine Unterstützung physiologischer bakterieller Abwehrsysteme, wie zum Beispiel des Laktoperoxidasesystems des Speichels (Tenovuo et al.: Relationship of the human salivary peroxidase system to oral health; J orla Path 13: 573-584, 1984), einen positiven Effekt auf die Mundgesundheit haben kann. Da nach Untersuchungen von Pruitt KM et al.: Limiting factors of the generation of hypothiocyanite ion, an antimicrobial agent, in human saliva; Caries Res. 16: 315-323, 1982; die Bioverfügbarkeit von Wasserstoffperoxid im Speichel die in-vivo Effektivität des Lactoperoxidasesystems limitiert, wurden Mittel inauguriert, die Enzyme enthalten, die Wasserstoffperoxid im Speichel bilden sollen. Diese System weisen folgende Nachteile auf:
- keine direkte Bereitstellung von Wasserstoffperoxid,
- bei hemmenden Einflußfaktoren in der Mundhöhle auf die Wasserstoffperoxid bildenden Enzyme kann die Bildung von Wasserstoffperoxid nicht im gewünsch ten Ausmaß zustande kommen,
- Behinderung physiologischer Penetrationsprozesse im Gewebe durch die Wasserstoffperoxidbildung an den Gewebegrenzflächen.

Aufgabe der Erfindung ist es, Erkrankungen in der Mundhöhle, insbesondere bakteriell bedingte entzündliche Erkrankungen zu verhindern, zu heilen oder zu lindern. Vornehmlich soll eine Schädigung der physiologischen Bakterienflora des Magen-Darm-Trakts und der Mundhöhle sowie eine Gefährdung durch Arzneimittel-Nebenwirkungen vermieden werden, in dem auf den Einsatz unphysiologischer xenobiotischer Wirkstoffe, zum Beispiel Antibiotika, plaquehemmende Wirkstoffe, Antiphlogistika verzichtet wird.

Der Erfindung liegt somit die Aufgabe zugrunde, neue wirksame Mittel zur Prophylaxe, Therapie und Linderung von Erkrankungen der Mundschleimhaut und des Zahnhalteapparates zu entwickeln, die bei Anwendung physiologischer Verbindungen eine gezielte Förderung physiologischer antibakterieller Abwehrmechanismen, physiologischer Heil- und Reparaturvorgänge sowie physiologischer Entgiftungsmechanismen in der Mundhöhle bewirken und ungefährlich in der Anwendung sind.

In einer ersten Ausführungsform der Erfindung wird die vorgenannte Aufgabe gelöst durch Mittel zur Förderung der Mundhygiene und der Mundgesundheit umfassend ionisch gebundene oder freie Thiocyanationen in einer Menge von 0,3 bis 30 g/kg und ein Hamstoff-Wasserstoff-Addukt (Carbamid-Perhydrat) im Verhältnis von 1 : 1 in einer Menge von 0,3 bis 30 g/kg Carbamid-Perhydrat neben an sich bekannten Hilfs- und Trägerstoffen.

Bei Thiocyanaten handelt es sich um ein physiologisches Substrat, das im Speichel natürlich vorkommt und in den anzuwendenden Mengen für Mensch und Tier ungefährlich ist. Carbamid-Perhydrat ist ein Harnstoff-Wasserstoffperoxid Addukt im Verhältnis 1 : 1 und stellt somit eine Kombination von zwei physiologischen Wirkstoffen dar.

Besonders bevorzugt im Sinne der vorliegenden Erfindung liegen die Thiocyanationen in Form von Alkalimetallsalzen einschließlich der Ammoniumsalze und ihrer Derivate vor. Hierbei handelt es sich somit um dissoziationsfähige Thiocyanate im Gegensatz zu organischen, kovalent gebundenen Thiocyanaten.

Besonders bevorzugt im Sinne der vorliegenden Erfindung sind die Alkalimetallsalze der Thiocyanate, ausgewählt aus Natrium-, Kalium- und Ammoniumthiocyanat.

Im Sinne der vorliegenden Erfindung enthalten die Mittel 0,3 bis 30 g/kg Thiocyanationen.

Die Anwendung von Carbamid-Perhydrat als direkter Wasserstoffperoxidbildner anstelle von enzymatisch gebildetem Wasserstoffperoxid bietet folgende Vorteile:
- Wasserstoffperoxidbereitstellung durch direkte Abspaltung anstelle eines Umwegs über störanfällige biochemische Bildung,
- Verbesserung der Wirkstoffpenetration durch den aus Carbamid-Perhydrat abgespalteten Harnstoff (Wohlrab W.: Bedeutung von Harnstoff in der externen Therapie; Hautarzt 40, Suppl. 9: 1981).

Besonders bevorzugt im Sinne der vorliegenden Erfindung wird das Gewichtsverhältnis von Thiocyanationen zu Carbamid-Perhydrat im Bereich von 10 : 1 bis 1 : 10, insbesondere 5 : 1 bis 1 : 5 eingestellt.

Die erfindungsgemäße Kombination von Thiocyanat im erfindungsgemäßen Konzentrationsbereich mit Carbamid-Perhydrat führte zu überraschenden neuartigen Effekten, die bei Anwendung der Einzelwirkstoffe oder bei niedrigerer Thiocyanatkonzentration in der Kombination nicht erkennbar waren. Diese neuartige Wirkungsqualität konnte klinisch signifikant gesichert werden, nachgewiesen über die Parameter Sulcus-Blutungs-lndex (Mühlemann HR & Son S: Gingival sulcus bleeding - a leading Symptom in initial gingivitis. Helv odont Acta 15: 107-11, 1973), Plaque-Index (Silness, J. & Löe, H.: Periodontal desease in pregnance: Correlation between oral hygiene and periodontal condition. Acta odont Scand 22: 121-135, 1964) und Messung der Gingivalflüssigkeitsmengen (Periotron 6000).

Neben den Wirkstoffen Thiocyanat und Carbamid-Perhydrat enthalten die erfindungsgemäßen Mittel insbesondere als Hilfs- und Trägerstoffe Putzkörper, Feuchthaltemittel, Bindemittel, Verdickungsmittel, Konservierungsstoffe, Fluoridverbindungen, Schäumer, Tenside, Süßungsmittel, Farbstoffe und/oder Aromatisierungsmittel. Beispielhaft seien hier Hydroxyethylcellulose, Methylparabene, Saccharin, Menthol, Pfefferminzöl und/oder Aqua dest. genannt.

Die erfindungsgemäßen Mittel können insbesondere in verschiedenen Formulierungen zur Anwendung kommen. Pasten, Gele, Mundwasser, Suspensionen, Sprays, Granulate, Pulver und Kaugummis, wobei die Thiocyanationen und das Carbamid-Perhydrat vorzugsweise in getrennter Anordnung, beispielsweise in getrennten Kompartimenten, bereitgestellt werden, so daß ein Vermischen erst unmittelbar vor oder während der Applikation stattfindet.

Die Applikation der erfindungsgemäßen Mittel kann mit einer Vielzahl von Techniken erfolgen, wobei eine Anwendung als Zahncreme oder Zahngel bei der täglichen häuslichen Zahn- und Mundpflege sowie eine Anwendung als Mundspülung und Mundwasser in Verbindung mit oder zur täglichen häuslichen Mundpflege besonders effektiv ist. Bei besonderen Indikationen sind spezielle Applikationen durch Zahnärzte oder zahnärztliches Personal vorgesehen, beispielsweise der Behandlung von Taschen am Zahnhalteapparat.

### Anwendungsbeispiel 1:

Thiocyanat (SCN⁻) und Carbamid-Perhydrat (CPH) wurde in Zahlpflegegelen mit üblichen Hilfs- und Trägerstoffen in verschiedenen Kombinationen und Konzentrationen in einer randomisierten, doppelblinden und Placebo-kontrollierten klinischen Studie auf die Hemmung der Plaqueakkumulation und auf Beeinflussung des Gesundheitszustands der Gingiva untersucht. 140 Probanden in 6 Untersuchungsgruppen benutzten die Zahnpflegegele über einen Zeitraum von 8 Wochen.

**Tabelle 1:**

| **Gruppe** | **SCN**^{**-**} | **CPH** |
|---|---|---|
| Gruppe A (Placebo)* n = 40 | 0 | 0 |
| Gruppe B* n = 20 | 0,1 Gew.-% | 0 |
| Gruppe C* n = 20 | 0,5 Gew.-% | 0 |
| Gruppe D n = 20 | 0,1 Gew.-% | 0,1 Gew.-% |
| Gruppe E n = 20 | 0,5 Gew.-% | 0,1 Gew.-% |
| Gruppe F* n = 20 | 0 | 0,1 Gew.-% |

| | | |
|---|---|---|
| *Vergleich | | |

Sowohl Thiocyanat allein (Gruppe B und C) als auch Carbamid-Perhydrat (Gruppe E) hatten keinen Einfluß auf Plaque und Gingivitis (Tabelle 3 und 4). Ebenso war die Kombination 0,1 Gew.-% Carbamid-Perhydrat mit der vergleichweise geringen Thiocyanatkonzentration von 0,1 Gew.-% (Gruppe D) ohne Einfluß auf die untersuchten Parameter (Tabelle 2 - 4). Im Unterschied dazu führte die Kombination 0,1 Gew.-% Carbamid-Perhydrat mit der fünffachen Thiocyanatkonzentration (0,5 Gew.-%) (Gruppe E) zu einer statistisch signifikanten Reduzierung der Plaque und der beiden Gingivitisparameter (Tabelle 2 - 4).

**Tabelle 2:**

| Sulcus-Fluid-Fließrate (Periotron 6000) in Gruppen mit unterschiedlichen SCN⁻/CPH Zahnpflegegelen | | | | |
|---|---|---|---|---|
| **Gruppe/Untersuchungstag** | **Erstuntersuchung** | **1 Woche** | **4 Wochen** | **8 Wochen** |
| Gruppe A n = 36 | 23,4 ± 13,9 | 22,2 ± 14,7 | 20,8 ± 14,6 | 18,6 ± 12,9 |
| Gruppe B n = 17 | 23 ± 9,8 | 22,5 ± 12,6 | 20,5 ± 10,5 | 20,4 ± 13,2 |
| Gruppe C n = 16 | 27,5 ± 19,6 | 25 ± 16,5 | 16,4 ± 10,7²⁾ | 25,9 ± 15,6 |
| Gruppe D n = 15 | 23,7 ± 15,9 | 19,7 ± 12 | 14,1 ± 12,3 | 24,2 ± 13,8 |
| Gruppe E n = 16 | 22,5 ± 14,8 | 17,5 ± 11,9 | 15,5 ± 10,4 | 11,8 ± 8,1³⁾ |
| Gruppe F n = 18 | 23 ± 14,5 | 23,5 ± 10,9 | 16,1 ± 7,8¹⁾ | 12,1 ± 8,2³⁾ |

| | | | | |
|---|---|---|---|---|
| Signifikanz der Mittelwertabweichung vom Mittelwert der Erstuntersuchung: ¹⁾ P = 0,90 | | | | |
| ²⁾ P = 0,95 | | | | |
| ³⁾ P = 0,99 | | | | |

**Tabelle 3:**

| Plaque-Index in Gruppen mit unterschiedlichen SCN⁻/CPH Zahnpflegegelen | | | | |
|---|---|---|---|---|
| **Gruppe/Untersuchungstag** | **Erstuntersuchung** | **1 Woche** | **4 Wochen** | **8 Wochen** |
| Gruppe A n = 36 | 0,336 ± 0,26 | 0,345 ± 0,27 | 0,339 ± 0,19 | 0,36 ± 0,24 |
| Gruppe B n = 17 | 0,383 ± 0,23 | 0,416 ± 0,29 | 0,45 ± 0,48 | 0,505 ± 0,35 |
| Gruppe C n = 16 | 0,444 ± 0,37 | 0,388 ± 0,28 | 0,447 ± 0,41 | 0,419 ± 0,28 |
| Gruppe D n = 15 | 0,355 ± 0,34 | 0,283 ± 0,22 | 0,307 ± 0,21 | 0,355 ± 0,22 |
| Gruppe E n = 16 | 0,452 ± 0,29 | 0,338 ± 0,26 | 0,309 ± 0,25 | 0,277 ± 0,29¹⁾ |
| Gruppe F n = 18 | 0,438 ± 0,24 | 0,372 ± 0,30 | 0,345 ± 0,2 | 0,295 ± 0,32 |

| | | | | |
|---|---|---|---|---|
| Signifikanz der Mittelwertabweichung vom Mittelwert der Erstuntersuchung: ¹⁾ P = 0,90 ²⁾ P = 0,95 ³⁾ P = 0,99 | | | | |

**Tabelle 4:**

| Sulcus-Blutungs-Index in Gruppen mit unterschiedlichen SCN⁻/CPH Zahnpflegegelen | | | | |
|---|---|---|---|---|
| **Gruppe/Untersuchungstag** | **Erstuntersuchung** | **1 Woche** | **4 Wochen** | **8 Wochen** |
| Gruppe A n = 36 | 1,083 ± 0,37 | 1,007 ± 0,33 | 1,073 ± 0,41 | 1,040 ± 0,52 |
| Gruppe B n = 17 | 1,298 ± 0,58 | 1,175 ± 0,59 | 1,227 ± 0,72 | 1,219 ± 0,68 |
| Gruppe C n = 16 | 1,112 ± 0,46 | 1,051 ± 0,55 | 1,057 ± 0,57 | 1,14 ± 0,58 |
| Gruppe D n = 15 | 1,005 ± 0,34 | 0,872 ± 0,35 | 0,995 ± 0,27 | 0,993 ± 0,37 |
| Gruppe E n = 16 | 1,129 ± 0,38 | 0,963 ± 0,43 | 0,904 ± 0,41. | 0,818 ± 0,36²⁾ |
| Gruppe F n = 18 | 1,266 ± 0,48 | 1,234 ± 0,42 | 1,117 ± 0,29 | 1,114 ± 0,53 |

| | | | | |
|---|---|---|---|---|
| Signifikanz der Mittelwertabweichung vom Mittelwert der Erstuntersuchung: ¹⁾ P = 0,90 ²⁾ P = 0,95 ³⁾ P = 0,99 | | | | |

## Patentansprüche

1. Mittel zur Förderung der Mundhygiene und der Mundgesundheit umfassend ionisch gebundene oder freie Thiocyanationen in einer Menge von 0,3 bis 30 g/kg und ein Harnstoff-Wasserstoffperoxid-Addukt (Carbamid-Perhydrat) im Verhältnis von 1 : 1 in einer Menge von 0,3 bis 30 g/kg Carbamid-Perhydrat neben an sich bekannten Hilfs- und Trägerstoffen.

2. Mittel nach Anspruch 1, **dadurch gekennzeichnet, dass** die Thiocyanationen in Form von Alkalimetallsalzen, einschließlich Ammoniumsalzen und ihrer Derivate vorliegen.

3. Mittel nach Anspruch 2, **dadurch gekennzeichnet, dass** die Alkalimetallsalze der Thiocyanate ausgewählt sind aus Natrium-, Kalium- und Ammoniumthiocyanat.

4. Mittel nach Anspruch 1, **dadurch gekennzeichnet, dass** das Gewichtsverhältnis von Thiocyanationen zu Carbamid-Perhydrat im Bereich von 10 : 1 bis 1 : 10 umfasst.

5. Mittel nach Anspruch 1, **dadurch gekennzeichnet, dass** das Gewichtsverhältnis von Thiocyanationen zu Carbamid-Perhydrat im Bereich von 5 : 1 bis 1 : 5 umfasst.

6. Mittel nach Anspruch 1, **dadurch gekennzeichnet, dass** die Hilfs- und Trägerstoffe Putzkörper, Feuchthaltemittel, Bindemittel, Verdickungsmittel, Konservierungsstoffe, Fluoridverbindungen, Schäumer, Tenside, Süßungsmittel, Farbstoffe und/oder Aromatisierungsmittel umfassen.

7. Mittel nach Anspruch 1, umfassend Pasten, Gele, Mundwasser, Suspensionen, Sprays, Granulate, Pulver und Kaugummis.

8. Mittel nach einem oder mehreren der Ansprüche 1 bis 7, zur Hemmung der Akkumulation bakterieller Plaque auf Zähnen, Zahnersatz und den angrenzenden Oberflächen, zur Prophylaxe, Therapie und Linderung von Entzündungen und Erkrankungen der Mundschleimhaut, der Gingiva, des Parodontiums und/oder zur Kariesprophylaxe.

9. Mittel nach einem oder mehreren der Ansprüche 1 bis 8, **dadurch gekennzeichnet, dass** die Thiocyanationen und Carbamid-Perhydrat in räumlich getrennter Anordnung vorliegen.

10. Verwendung eines Mittels wie in einem oder mehreren der Ansprüche 1 bis 9 definiert, zur Herstellung eines Therapeutikums zur Hemmung der Akkumulation bakterieller Plaque auf Zähnen, Zahnersatz und den angrenzenden Oberflächen, zur Prophylaxe, Therapie und Linderung von Entzündungen und Erkrankungen der Mundschleimhaut, der Gingiva, des Parodontiums und/oder zur Kariesprophylaxe.

## Claims

1. Agents for the promotion of oral hygiene and oral health, comprising ionically bound or free thiocyanate ions in an amount of from 0.3 to 30 g/kg and a 1:1 urea/hydrogen peroxide adduct (carbamide perhydrate) in an amount of from 0.3 to 30 g/kg of carbamide perhydrate in addition to per se known additives and vehicles.

2. The agents according to claim 1, **characterized in that** said thiocyanate ions are present in the form of alkali metal salts including ammonium salts and their derivatives.

3. The agents according to claim 2, **characterized in that** said alkali metal salts of thiocyanates are selected from sodium, potassium and ammonium thiocyanates.

4. The agents according to claim 1, **characterized in that** the weight ratio of thiocyanate ions to carbamide perhydrate is within a range of from 10:1 to 1:10.

5. The agents according to claim 1, **characterized in that** the weight ratio of thiocyanate ions to carbamide perhydrate is within a range of from 5:1 to 1:5.

6. The agents according to claim 1, **characterized in that** said additives and vehicles comprise cleaning particulates, humectants, binders, thickeners, preservatives, fluoride compounds, foaming agents, surfactants, sweeteners, colorants and/or flavoring agents.

7. The agents according to claim 1, comprising pastes, gels, mouth washes, suspensions, sprays, granules, powders and chewing gums.

8. The agents according to one or more of claims 1 to 7 for the inhibition of bacterial plaque accumulation on teeth, dental prostheses and adjacent surfaces, for the prophylaxis, therapy and alleviation of inflammations and diseases of the oral mucosa, gingiva, periodontium, and/or for caries prophylaxis.

9. The agents according to one or more of claims 1 to 8, **characterized in that** said thiocyanate ions and carbamide perhydrate are present in a spatially separated configuration.

10. Use of an agent as defined in one or more of claims 1 to 9 for the preparation of a therapeutic agent for the inhibition of bacterial plaque accumulation on teeth, dental prostheses and adjacent surfaces, for the prophylaxis, therapy and alleviation of inflammations and diseases of the oral mucosa, gingiva, periodontium, and/or for caries prophylaxis.

## Revendications

1. Produit pour favoriser l'hygiène buccale et la santé buccale, comprenant des ions thiocyanate libres ou liés d'une manière ionique, en une quantité de 0,3 à 30 g/kg, et un produit d'addition urée-peroxyde d'hydrogène (carbamide-perhydrate) selon un rapport de 1 : 1, en une quantité de 0,3 à 30 g/kg de carbamide-perhydrate, en plus d'adjuvants et supports connus en soi.

2. Produit selon la revendication 1, **caractérisé en ce que** les ions thiocyanate se présentent sous forme de sels de métaux alcalins, y compris les sels d'ammonium et les dérivés de ceux-ci.

3. Produit selon la revendication 2, **caractérisé en ce que** les sels de métaux alcalins des thiocyanates sont choisis parmi les thiocyanates de sodium, de potassium et d'ammonium.

4. Produit selon la revendication 1, **caractérisé en ce que** le rapport en poids des ions thiocyanate au carbamide-perhydrate est compris dans la plage de 10:1 à 1:10.

5. Produit selon la revendication 1, **caractérisé en ce que** le rapport en poids des ions thiocyanate au carbamide-perhydrate est compris dans la plage de 5:1 à 1:5.

6. Produit selon la revendication 1, **caractérisé en ce que** les adjuvants et supports comprennent des agents nettoyants, des humectants, des liants, des épaississants, des conservateurs, des composés à base de fluorure, des agents moussants, des tensioactifs, des édulcorants, des colorants et/ou des agents aromatisants.

7. Produit selon la revendication 1, qui englobe les pâtes, les gels, les bains de bouche, les suspensions, les sprays, les granulés, les poudres et les gommes à mâcher.

8. Produit selon une ou plusieurs des revendications 1 à 7, pour empêcher l'accumulation de la plaque bactérienne sur les dents, les prothèses dentaires et les surfaces contiguës, pour la prophylaxie, le traitement et l'atténuation des inflammations et maladies de la muqueuse buccale, des gencives, du parodonte et/ou pour la prophylaxie des caries.

9. Produit selon une ou plusieurs des revendications 1 à 8, **caractérisé en ce que** les ions thiocyanate et le carbamide-perhydrate sont présents selon une disposition spatialement distincte.

10. Utilisation d'un produit selon une ou plusieurs des revendications 1 à 9 pour préparer un agent thérapeutique pour empêcher l'accumulation de la plaque bactérienne sur les dents, les prothèses dentaires et les surfaces contiguës, pour la prophylaxie, le traitement et l'atténuation des inflammations et maladies de la muqueuse buccale, des gencives, du parodonte et/ou pour la prophylaxie des caries.
